# EUROPEAN PATENT SPECIFICATION

(11) **EP 1 868 503 B1**
(45) Date of publication and mention of the grant of the patent: **14.11.2012**
(21) Application number: 06747420.5
(22) Date of filing: 21.04.2006
(51) Int. Cl.: A61B 5/145, A61B 5/151

(54) **GLUCOSE METER WITH ER:YAG LASER LANCING DEVICE**
GLUCOSEMESSGERÄT MIT ER:YAG-LASERSTECHVORRICHTUNG
GLYCOMÈTRE AVEC DISPOSITIF DE PERFORATION À LASER ER:YAG

(30) Priority: 14.04.2006 KR 20060034060
(43) Date of publication of application: 26.12.2007
(73) Proprietor: Isotech Co., Ltd, Yuseong-gu, Daejeon 305-701 (KR)
(72) Inventor: CHOI, Kee Jung, Daejeon 375-769 (KR); WOO, Hwa Je, Daejeon 302-210 (KR); POLUSHKIN, Valery, G., Moscow Region, 142-190 (RU)
(74) Representative: Marshall, John Grahame
(86) International application number: PCT/KR2006/001511
(87) International publication number: WO 2007/119900

(56) References cited:
- EP-A- 1 048 310
- JP-A- 7 039 542
- US-A- 5 643 252
- US-A- 5 947 957
- US-A- 5 947 957
- US-A- 5 993 439
- US-A1- 2002 058 953
- US-A1- 2002 173 732
- US-A1- 2003 149 427
- US-B1- 6 206 841
- US-B1- 6 332 871

## Description

### [Technical Field]

The present invention relates to a painless blood lancing device using the laser and a blood glucose meter using the same, especially, to a new technology for use in the blood glucose monitoring field, which is capable of carrying out the lancing and blood glucose measuring by means of a single equipment. More particularly, the present invention relates to a personal portable painless lancing device, which is capable of lancing from the skin without any pain by using an Er:YAG laser technology in the medical field, and further by using a laser beam having a wavelength of 2.94*µ*m, which has a strong absorption force with respect to water so that it does not harm the human body, and a blood glucose meter using the same.

### [Background Art]

In general, an Er:YAG laser beam-generating device (laser head) used for lancing without any pain comprises a part called a reflector in the outer shape, which is made by depositing or plating Ag/Ni/Cu or alloy thereof on a surface of a metal body several times and machining the surface thereof, and a flash lamp for pumping the laser beam and an Er:YAG crystal are built in the reflector. The strength of the laser beam radiated from the Er:YAG laser beam-generating device varies based on the radiation amount of the flash lamp and the volume, size and the density of the Er:YAG crystal.

However, the conventional painless lancing device using a laser was not good at adhesion property between the skin and the Er:YAG laser beam-generating device, so that there occurred problems that sound such as "flump" was produced when the skin was punched by the laser beam, a white spot was produced at the skin from which the blood had been collected, and smoke produced from the burned skin was blown out to thereby make the mind of the lanced patient uncomfortable. Also, the retina can be injured due to strong stimulation, because a flint of the flash lamp and the laser beam radiated at the skin were in view.

Thus, the conventional painless lancing device using the Er:YAG laser beam-generating section resulted in the shrinkage of the intention for use it due to the fear complex about the lancing, despite the advantage that it could lance without any pain in contrast to a needle shaped lancet.

Reviewing the technical field of art using a strip for measuring the blood glucose, three major companies such as Johnson & Johnson, Abbot in the US and Roche of Deutschland have commanded the market in the world, and Allmedicus, Infopia, i-sense, and the like in Korea have put the equipment for measuring the blood glucose after lancing by means of the needle shaped lancet in the market.

In case of an insulin-dependent patient who should check the blood glucose in real time for two or three times a day, the lancing is usually carried out by piercing the end portion of a finger by means of a needle shaped lancet. However, this lancing method causes big pains in addition to the lancing beyond the necessary amount of blood, because the lancing is carried out by the incision of the skin tissue by means of the lancet. Moreover, while it is necessary to stop bleeding of a portion from which the blood has been collected, the incision portion will not be cured quickly after the stop of the bleeding, so that there is existed the possibility of infection due to virus or germs.

Furthermore, while there has been recently disclosed an infrared-ray scanning type blood glucose meter for indirectly measuring the blood glucose, it is not widely used because the reliability of the measured value is relatively degraded as compared to the direct lancing method.

Meanwhile, it was inconvenient to purchase the equipment and maintain it because the lancing device and the blood glucose meter were separately manufactured and provided conventionally so that the measuring of the blood glucose could be performed only after the both equipment are all prepared.

A portable blood sampling device is disclosed by US 5947957 which uses a laser to puncture the skin.

### [Disclosure]

### [Technical Problem]

Accordingly, the present invention was devised to solve such conventional problems, and it is an object of the present invention to provide a painless laser lancing device, which is not exposed to flint or smoke produced from the burning of the skin as the adhesion property between the skin and the Er:YAG laser beam-generating section is excellent, the lancing amount for one time is very small, and no pain was caused in lancing.

Another object of the present invention is to provide a personal portable blood glucose meter made by incorporating the painless lancing device and the blood glucose measuring device into a unitary equipment.

### [Technical Solution]

To achieve the above objects, according to the present invention, the painless minimum lancing device for collecting the blood by using an Er:YAG laser according to the present invention concentrates the laser beam on the skin locally and enhances the adhesion property between the skin and the laser beam-generating section to thereby overcome the psychological rejection symptom of the lanced patient.

In this regard, the painless minimum lancing device for collecting the blood by using an Er:YAG laser according to the present invention is characterized by comprising an Er:YAG laser beam-generating section for generating the laser beam required to punch skin; an adapter mounted at the front end of the Er:YAG laser beam-generating section so that the laser beam can be concentrated on the skin locally; and a case for accommodating the Er:YAG laser beam-generating section and the adapter.

### [Advantageous Effects]

As described above, the present invention can prevent the infection from the virus or the germs without separate sterilization of the lancing portion, since the radiated laser beam instantly heats the skin to above about 1000°C to thereby perform the sterilization.

Furthermore, it is possible to primarily remove the smell and smoke produced from the burning of the skin and the occurrence of dull noise by disposing the adapter and the skin adhesion cap having good adhesion property with the skin at the front end of the Er:YAG laser beam-generating section.

Also, it is possible to intercept the exposure of the flash to the outside by means of the case made of engineering plastic.

### [Description of Drawings]

FIG. 1 is a model view showing the basic structure of a painless minimum lancing device using an Er:YAG laser according to the present invention;

FIG. 2 is a model view showing a minimum painless lancing device using an Er:YAG laser according to an embodiment of the present invention;

FIG. 3 is a model view showing a skin adhesion cap used in an embodiment of the present invention;

FIG. 4 is a solid model view showing the skin adhesion cap of FIG. 3 taken from the rear side;

FIG. 5 is an exploded perspective view showing an example of accommodating an adapter and an interlock switch at one side of the case of the present invention;

FIG. 6 is a view showing an inner status of the painless minimum lancing device using an Er:YAG laser according to an embodiment of the present invention, by opening one side of the case;

FIG. 7 is a view showing an inner construction of the painless lancing device by removing a part of the case material at a connection portion between the skin adhesion cap shown in FIG. 6 and the Er:YAG laser beam-generating section;

FIG. 8 is a construction view showing a blood glucose meter according to an embodiment of the present invention.

[Explanation of the drawing numeral]

1: front surface securing cover

2: reflector

3 : flash lamp

4: rear surface securing cover

5 : focusing lens

6 : focusing lens holder

7: semi-permeable mirror

8: front surface securing holder

9: Er:YAG crystal

10: rear surface securing holder

11: total reflection mirror

12 : inspection film

13: skin adhesion cap

14: connection device

15 : buffer spring

16: support jaw

21: capacitor

22: charging transformer

23: blood glucose measuring circuit

24: blood glucose sensor strip

25: connector

26: input and output device

> 27: triggering transformer

28: laser beam control circuit

29: electric power supply source (charging battery)

30: MPU and peripheral circuit

31: interlock switch operating handle

32: case

33: interlock switch securing holder

### [Mode for Invention]

The painless minimum lancing device to settle the technical task of the present invention is characterized by claim 1.

More particularly, as described in claim 1 of the present invention, the lancing device can comprise an Er:YAG laser beam-generating section from which the laser beam (beam) is generated and radiated in a single direction; an adapter installed at the front end of the Er:YAG laser beam-generating section, and disposed in an axis identical with that of an Er:YAG crystal with a predetermined distance from the Er:YAG crystal constructing the Er:YAG laser beam-generating section; a skin adhesion cap installed at the front end of the adapter so that it can be in close contact with the skin, and is capable of preventing the contamination of the Er:YAG laser beam-generating section due to the blood gushed out at the time of the skin punch and the smoke produced from the burning of the skin, and confirming if it is reused by the naked eye; and a case for accommodating the Er:YAG laser beam-generating section and the adapter therein.

Also, the present invention comprises a body penetratingly formed internally to make the laser beam pass through, secured detachably to the front end of the adapter at a rear end portion thereof, and inclined by preferably 68° at a front end thereof with respect to the laser beam passing through the inside of the body so that it is in close contact with the skin; and an inspection film installed at the rear end of the body to enable inspection if the skin adhesion cap is used, to prevent the dispersion of the blood occurring at the time of the lancing and the contamination of the adapter due to smoke, and to remove the problem of infection of the patient due to reuse of the skin adhesion cap (claims 1 and 2).

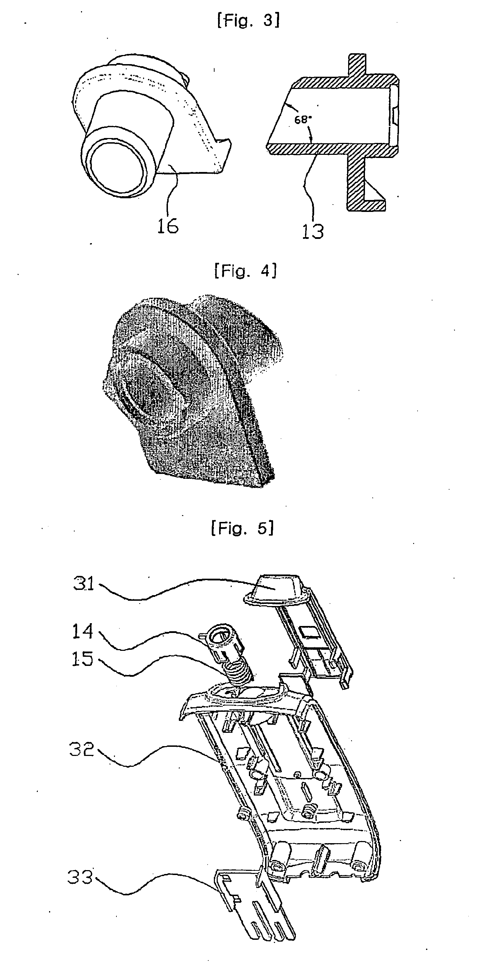

In this instance, the skin adhesion cap is preferably made from polyester, which is hot compressed with high strength, and the like, so that noise/flash light/smoke and the like arising at the time of the laser beam radiation can be intercepted, and is made in structure such that the skin can be a little pushed and entered into the front end of the body, when it is contacted with a lancing portion. Also, in respect of sanitation, the skin adhesion cap is constructed to be easily detachable from the front end of the adapter so that it can be disposable. In addition, more preferably, a support jaw can be formed at the outer surface of the body of the skin adhesion cap for preventing the disturbance with intercepting the passing through of the air when it is used (confer FIGs. 3 and 4).

In addition, the Er:YAG laser beam-generating section may preferably include a reflector having a receiving space formed at the inside;

an Er:YAG crystal disposed in the receiving space of the reflector;

a total reflection mirror installed at the rear surface of the Er:YAG crystal;

a semi-permeable mirror installed at the front end of the Er:YAG crystal;

a flash lamp installed at the inside of the receiving space in such a manner as to be spaced apart by a certain interval from the Er:YAG crystal;

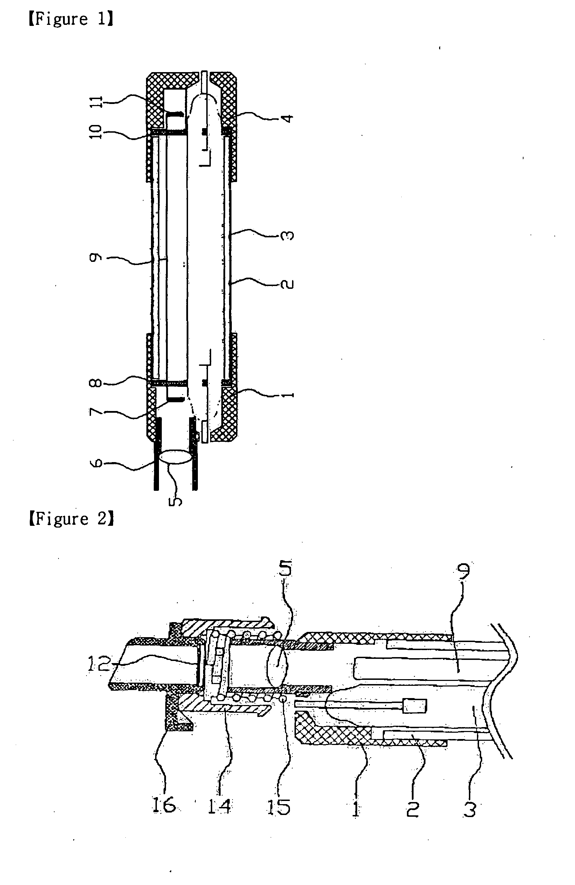

front and rear surface securing covers respectively installed at both front and rear ends of the reflector so that both ends of the Er:YAG crystal and the flash lamp can be supported; and

a front and rear surface securing holder inserted into the outside of both sides of the front and rear ends of the reflector for integrally securing the front and rear surface securing cover and the reflector (confer claim 3 and FIGs. 1, 2).

Moreover, the adapter may preferably include a focusing lens holder inserted into the front surface securing holder of the Er:YAG laser beam-generating section;

a focusing lens inserted into an inside passage of the focusing lens holder so that the laser beam can be concentrated on a skin punch portion to enable the painless lancing;

a connection device for the skin adhesion cap, which is installed at the front end of the focusing lens holder; and

a buffer spring interposed between the connection device and the focusing lens holder (claim 4).

According to the construction as described above, it is possible to prevent the contamination of the adapter and the focusing lens due to blood and smoke gushed out at the time of the lancing, by means of the contamination prevention function of the skin adhesion cap detachably mounted to the connection device, and it is possible to radically reduce the psychological pain and the real pain of the lanced patient because the skin injury of the lancing portion can be minimized. Also, the spring inserted between the adapter and the focusing lens holder performs buffer action when the disposable skin adhesion cap is contacted with the skin.

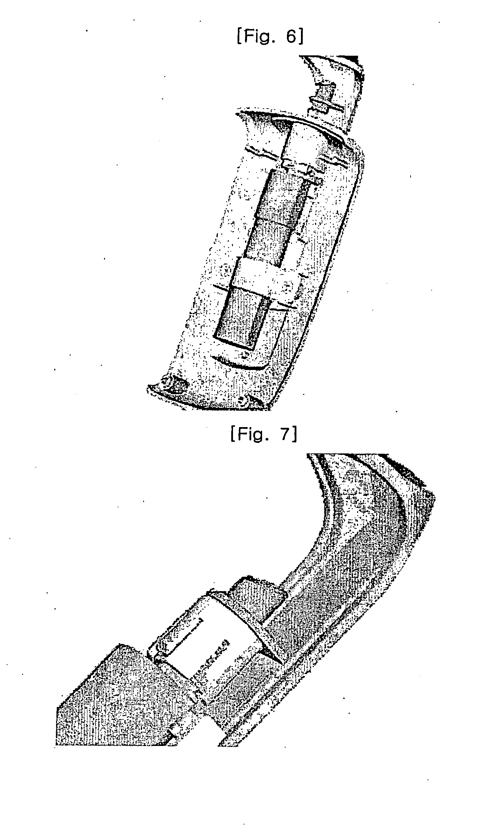

Moreover, with regard to the painless minimum lancing device using the Er:YAG laser of the present invention, as shown in FIGs. 5 through 7, since an interlock switch is mounted at the case to which the adapter is accommodated, the laser beam cannot be used for other object or use than the lancing.

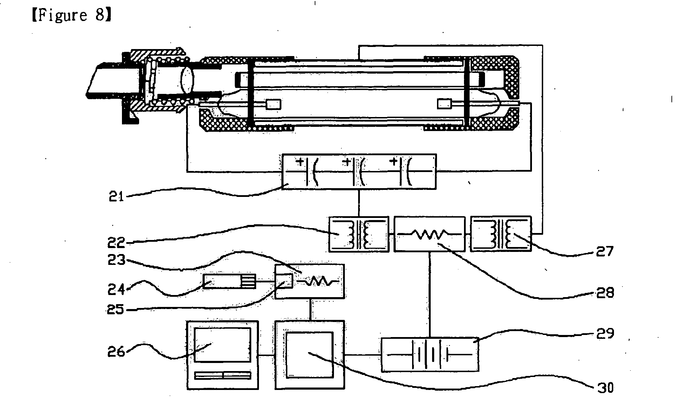

Furthermore, as described in claims 5 and 6, the blood glucose meter of the present invention comprises, in addition to the painless minimum lancing device using an Er:YAG laser as described above, a connector for inserting a blood glucose sensor strip, a blood glucose measuring circuit, and means for displaying measured blood glucose value including LCD, and the like, so that the lancing and the blood glucose measuring can be performed with a single equipment (confer FIG. 8). Also, it is preferable that a microprocessor is loaded on a PCB for controlling the operation of the respective constructional element, and a program for recording /managing past results of the blood glucose measuring is built therein.

In addition, the painless minimum lancing device using an Er:YAG laser and the blood glucose meter using the same require an input and output means for displaying the operation/management and status/result, and it is very easy for those skilled in the field of art to conceive it.

In other words, it has been well known in the field of art to which the present invention pertains, and in the field of electronic industry to construct the control method and device for the output energy of the laser beam, the control method and the device for focusing of the laser beam, means for displaying the status/result, and the like, within the scope of the technical spirit of the present invention. Thus, detailed referring and description of the input and output means are abridged in the present specification.

Hereinafter, the construction and action of the painless minimum lancing device using an Er:YAG laser and a blood glucose meter according to an embodiment of the present invention will be described in detail with reference to the appended drawings.

1. A painless minimum lancing device using an Er:YAG laser

FIGs. 1 through 4 are model views showing a painless lancing device using a laser according to a preferred embodiment of the present invention, an adapter becoming one element of the primary constructional elements, and a disposable skin adhesion cap.

As shown in FIG. 1, an Er:YAG laser beam-generating section of the present embodiment comprises a reflector 2 having a receiving space formed at the inside, an Er:YAG crystal 9 inserted into the receiving space of the reflector 2 so that both ends thereof are exposed to the outside of the receiving space, mirrors 7, 11 secured at both ends of the Er:YAG crystal 9, and a flash lamp 3 inserted into the receiving space of the reflector with maintaining a predetermined distance from the Er:YAG crystal 9. Both ends of the Er:YAG crystal and the flash lamp are supported by a front surface securing cover 1 and a rear surface securing cover 4 installed respectively at both ends of the reflector so that they cannot move, and front and rear surface securing holders 8, 10 are respectively inserted outwardly at front and rear both ends of the reflector for integrally securing the front and rear surface securing covers and the reflector.

Herein, an semi-permeable mirror 7 is installed at the front of the Er:YAG crystal 9, and a total reflection mirror 11 is installed at the rear side.

The Er:YAG crystal 9 functions to produce a laser beam strong to hydrophile property and having a wavelength of 2.94µm of 0.1-0.6mJ by means of lasing transition 4I11/2 → 4I13/2 of the crystal after inducing and focusing flash radiated from the flash lamp 3, and the strength of the laser beam is determined based on the crystallization structure, density, volume of the crystal, and the light emitting amount of the flash lamp.

In the present embodiment, the crystal is made by forming by doping fine Yttrium Aluminum Garnet (Y₃A₁₅G₁₂) powder at the melting temperature of 1870°C so that the laser beam can be radiated uniformly in a predetermined direction and concurrently the strength can be highly maintained, performing surface treatment with OH-free special coating, and then precisely machining the resultant product with a surface roughness below 1µm.

Herein, since the Er:YAG crystal 9 should be maintained at a clear and transparent status in the crystallization structure, it takes substantially long time in doping, and it requires know-how of the skilled technical manpower because minute shear machining and grinding machining below 1*µ*m are required to maintain the linearity of the laser beam (beam) and to remove interference and scattering.

The flash lamp 3 for pumping the laser beam is a device for producing the flash of about 80∼100WS so as to supply energy to the Er:YAG crystal 9. In the present embodiment, it is induced to discharge the flash of about 80∼100WS level by using the trigger voltage, after filling a cylindrical stick type glass tube under the vacuum state with xenon gas of 400∼700 mmHg and applying a high voltage at both electrodes installed with an interval of about 40∼45mm there-between.

The reflector made by installing the Er:YAG crystal 9 and the flash lamp 3 with a predetermined interval is an element for forming outer appearance of the Er:YAG laser beam-generating section, and functions to induce and focus the flash light, which is emitted from the flash lamp 3 and reflected at the inner surface of the reflector not less than 98%, to the Er:YAG crystal 9.

In the present embodiment, primarily silver is vapor deposited on the surface of the quartz glass to thereby maintain the reflection ratio not less than 98% at the time of manufacturing the reflector, and performing the coating of an alloy made of nickel and copper to prevent the oxidation of the silver (Ag), and then performing the milling of the surface thereof.

The mirrors denoted by the numerals 7 and 11 are elements for making the laser beam focusd by the Er:YAG crystal 9 be radiated with maintaining the linearity in the predetermined direction, then they are machined minutely so that the flat degree of 10⁻⁹m can be maintained according to the radiation angle and the direction of the laser beam, and the surface of them are made smooth by the polishing.

Also, the semi-permeable mirror 7 installed at the direction (front end) in which the laser beam is radiated, is machined so that permeation ratio can be maintained above 50%, and the total reflection mirror 11 installed at the opposing side (rear side) is machined so that the laser beam focusd to the Er:YAG crystal can be reflected in the direction in which they are radiated not less than 90% to thereby maintain the linearity and contrive to induce the radiating direction.

This is achieved by improving the conventional Er:YAG laser technology, and resulted in the minimization of the laser beam loss due to interference and scattering and improvement in the permeation degree and the linearity of the laser beam by installing the total reflection /semi-permeable mirrors 7, 11 at both ends of the Er:YAG laser beam-generating section respectively.

As shown in FIGs. 1 and 2, the adapter of the present invention is installed at the front side of the total reflection mirror 7 of the Er:YAG laser beam-generating section.

Also, it comprises a focusing lens holder 6, a focusing lens 5 inserted into an inner passage of the focusing lens holder, a connection device 14 inserted into an outer surface of the focusing lens holder, and a buffer spring 15 interposed between them.

The focusing lens 5 is a construction element for focusing the laser beam radiated in the predetermined direction at the skin for lancing with an accurate focus, and the skin penetration depth is made to be not more than 0.5mm and the diameter is in the range of 0.3mm lest it should stimulate a nervous cell in the inner skin tissue to thereby cause pains, although it can punch a hole of 0.1∼0.5mm diameter to a depth of 0.2∼4mm. In order to maintain the skin penetration depth and the punch diameter, the distance from the total reflection mirror 7 in the direction to which the laser beam is radiated to the focusing lens is maintained to be in the range of 13∼15mm, and the distance from the focusing lens to the skin surface is adjusted to be in the range of 17.5∼18.0mm.

As a result, it is possible to easily collect the blood not less than 0.5µl required to measure the blood glucose by stimulating the blood flow of the capillary existing between the outer tissue and the inner tissue to be in the range of 400µ sec∼500µ sec, and minimize the size of the injury portion and the pain.

It is possible to adjust the lancing amount to be 1*µ*l∼500*µ*l according to the energy strength of the laser beam, and there is an advantage of primary preventing the occurrence of the smell produced from the burning of the skin and dull noise of "flump" at the time of the lancing. Also, according to the present invention, the energy loss due to the interference and scattering is minimized and the efficiency has been improved by performing the polishing to 10 m after minutely machining the surface of the focusing lens 5.

The adapter of the present embodiment as described above functions to intercept/absorb the flash light produced from the flash lamp and the noise produced from the lancing, which were the basic problems of the prior art.

In other words, the adapter of the present invention can be a kind of "airtight explosion-proof type" adapter.

In addition, the connection device 14 inserted into the front end of the focusing lens holder having a focusing lens built therein is made from compression polyester material to improve the effect of the noise interception through maintaining the airtight property.

Moreover, the periphery of it is sealed by means of a thermal contraction tube made of foamed compression type engineering plastic material to intercept the flash light radiated from the flash lamp 3 and the final noise.

The skin adhesion cap (confer FIGs. 3 and 4) denoted by the drawing numeral 13 is constructed to be easily inserted into the connection device 14 at the front end of the adapter when it is pressed by the finger.

The skin adhesion cap is preferable to be made of flexible soft material (for instance, silicon rubber) so that it can be contacted with a little compression state to maintain airtight property so as to easily collect the blood and remove the pain caused by the lancing.

Also, since the skin adhesion cap of the present embodiment is provided with a thin inspection film 12 made of polyethylene material at the rear cylindrical end thereof, which is inserted into the inside of the connection device 14 of the adapter and met with the focusing lens, it is possible to prevent the decrease of the linearity and the permeation ratio of the laser beam at the time of the punch of the skin with the Er:YAG laser beam due to the staining of the blood at the focusing lens, and prevent the contamination of the collected blood. In addition, since the polyethylene film is punched by the laser beam at the time of radiating the laser beam (that is, while it is used at least once), it is possible to simply confirm with the naked eye whether it can be used again. Moreover, a support jaw 16 is projected from the outer surface of the body of the skin adhesion cap for preventing the swing at the time of the lancing.

Furthermore, the painless minimum lancing device using the Er:YAG laser according to the present embodiment is provided with two safety devices.

One safety device of them is an interlock sensor (not shown in the drawing) installed at the connection device to continuously maintain the locking state unless the skin adhesion cap 13 is completely secured at the connection device 14 of the adapter. Thus, the interlock sensor is operated to radiate the laser beam only when the finger is pressed with being contacted with the skin adhesion cap to thereby be completely secured to the connection device.

The other is, as shown in FIG. 5, a construction in which the interlock sensor (not shown) is secured at an interlock switch securing holder 33 disposed at the inside of the case 32, and a stopper shaped handle 31 for operating the interlock switch, which is disposed at the outside of the case, is operated to radiate the laser beam. That is, these safety devices function so that the laser beam cannot be radiated on other portions of the human body than the finger.

2. Blood Glucose meter

FIG. 8 is a model view showing a blood glucose meter according to an embodiment of the present invention.

As shown in the drawing, the blood glucose meter is constructed by comprising total reflection/semi-permeable mirrors 7, 11 respectively installed at both ends of an Er:YAG crystal 9, and a PCB embodied by a laser beam control circuit 28 consisted of a high output capacitor 21 and minimal charging/triggering transformers 22, 27, so that it can be used at any time regardless of the place, to thereby achieve the miniaturization and the light-weight of the device.

Also, the blood glucose meter employs a power supply device 29 using a lithium-ion battery to enable the lancing and the blood glucose measuring above one hundred times without the supply of the AC electric power, and a peripheral circuit 30 including a microprocessor unit (MPU) is embodied into a small PCB.

The laser beam control circuit 28 embodied into a PCB charges voltage applied from the transformer to the capacitor and controls the amount of the electric power source by means of the control circuit, to thereby adjust the emitting amount of the flash lamp to be 80∼100WS, resulting in the control of the strength of the Er:YAG laser beam to be from 0.1J in the minimum to 0.6 J in the maximum.

In addition, the safety device for intercepting the laser beam from being radiated on a portion except the lancing portion, is coupled in movement to the display of [READY] of the input and output device 26 including the laser beam control circuit 28 and the LCD, so that it can be used only for the lancing and the blood glucose measuring of the finger.

The connector denoted by the drawing numeral 25 is a construction element for inserting a blood glucose sensor strip 24 with blood glucose information stored therein, and the blood glucose information stored in the blood glucose sensor strip is transferred to a blood glucose measuring circuit 23, which is embodied into a PCB, and displayed as numerical value through the LCD, and the measured blood glucose value is stored.

Herein, the blood glucose sensor strip is a strip for collecting the blood of 0.5µl by means of the method using the Er:YAG laser and providing the blood glucose information according to the electric chemical reactions, and comprises a blood insert absorbing the collected blood, an insert sensor for determining if it is possible to collect the blood glucose information from the absorbed blood, and a contact type connector 25 for transferring the collected blood glucose information to the blood glucose measuring circuit 23 by means of the electric chemical analysis method after reacting it with a reagent injected on the strip.

Accordingly, it is possible to accurately measure the blood glucose with minimum blood.

In the present embodiment, a 32bit MPU and a peripheral circuit are installed on the PCB and an operation software is built therein so as to control/perform the blood glucose measuring as described above, to thereby achieve the simplification and the miniaturization of the constructing circuit, so that it is possible to prevent the causing factors of the measuring error, occurrence of several disorders and erroneous action due to the complex circuit construction in advance.

[Modified embodiment]

Meanwhile, the blood glucose meter of the present invention comprises a control button in addition to the construction elements as described above, for varying the strength of the laser beam from eight stages in the minimum to sixteen stages in the maximum, by controlling the amount of the electricity supplied to the flash lamp according to the feature of the lancing people, by means of the voltage from 400V in the minimum to 700V in the maximum applied to an anode and a cathode.

Also, the blood glucose meter of the present embodiment has a desired software program installed therein for displaying the past blood glucose measuring data, remaining amount of the charging battery, and date and time of the blood glucose value, and the like, by means of numeric values or symbols in the LCD display.

### [Industrial Applicability]

As described above, the present invention collects the blood of about 0.5µl without any pain and use it in the blood glucose measuring, so that it will be very advantageous if the present device is supplied to the insulin-dependent patient (a diabetic).

Furthermore, the present invention can prevent the infection from the virus or the germs without separate sterilization of the lancing portion, since the radiated laser beam instantly heats the skin to above about 1000°C to thereby perform the sterilization.

Also, it is possible to primarily remove the smell and smoke from the burning of the skin and the occurrence of dull noise by disposing the adapter and the skin adhesion cap having good adhesion property with the skin at the front end of the Er:YAG laser beam-generating section.

Moreover, it is possible to intercept the exposure of the flash light to the outside by means of the case made of an engineering plastic.

## Claims

1. A painless minimum lancing device using an Er:YAG laser, comprising:
an Er:YAG laser beam-generating section (1-4,7-11) for generating a laser beam required to punch skin;
an adapter (5,6,14,15) installed at the front end of the Er:YAG laser beam-generating section so that the laser beam can be locally concentrated on a skin punch portion; and
a case (32) for accommodating the Er:YAG laser beam-generating section and the adapter therein,
further comprising a skin adhesion cap (13) detachably installed at the front end of the adapter and comprising:
a body having a hollow interior so that the laser beam can pass through, a rear end portion of the body being secured detachably to the front end of the adapter, and
a film (12) installed at the rear end of the body to prevent the dispersion of the blood occurring at the time of the lancing and the contamination of the adapter due to the smoke,
**characterised in that** the body has a fronted thereof being inclined and shaped so that it can be close contact with the skin; and the film (12) is an inspection film which is rupturable by the laser beam in use to provide a visual indication of when the skin adhesion cap has been used,
so as to remove the problem of infection of the patient due to reuse of the skin adhesion cap.

2. The painless minimum lancing device according to claim 1, wherein the front end portion of the body of the skin adhesion cap (13), which in use is in close contact with the skin, is inclined by 68° with respect to the laser beam passing through the inside of the body.

3. The painless minimum lancing device according to claim 1 or claim 2, wherein the Er:YAG laser beam-generating section (1-4,7-11) includes:
A) a reflector (2) having a receiving space formed at the inside;
B) an Er:YAG crystal (9) disposed in the receiving space of the reflector;
C) a total reflection mirror (11) installed at the rear surface of the Er:YAG crystal;
D) a semi-permeable mirror (7) installed at the front end of the Er:YAG crystal;
E) a flash lamp (3) installed at the inside of the receiving space in such a manner as to be spaced apart by a certain interval from the Er:YAG crystal;
F) front and rear surface securing covers (1,4) respectively installed at both front and rear ends of the reflector so that both ends of the Er:YAG crystal and the flash lamp can be supported; and
G) a front and rear surface securing holder (8,10) inserted into the outside of both sides of the front and rear ends of the reflector for integrally securing the front and rear surface securing cover and the reflector.

4. The painless minimum lancing device according to claim 1 or claim 2, wherein the adapter (5,6,14,15) includes:
A) a focusing lens holder (6) inserted at one end thereof into the front surface securing holder of the Er:YAG laser beam-generating section;
B) a focusing lens (5) inserted into an inside passage of the focusing lens holder so that the laser beam can be concentrated on a skin punch portion;
C) a connection device (14) installed at the front end of the focusing lens holder; and
D) a buffer spring (15) interposed between the connection device and the focusing lens holder.

5. A blood glucose meter comprising:
a minimum painless lancing device according to any one of claims I to 4;
a connector (25) for inserting a blood glucose sensor strip (24);
a blood glucose measuring circuit (23); and
means (26) for displaying measured blood glucose value.

6. The blood glucose meter according to claim 5, further including a program for recording/managing results of the blood glucose measuring installed in a microprocessor unit (MPU) (30).

## Patentansprüche

1. Vorrichtung für einen schmerzlosen Minimaleinstich unter Verwendung eines Er:YAG-Lasers mit folgenden Merkmalen:
einem einen Er:YAG-Laserstrahl erzeugenden Abschnitt (1-4, 7-11) zum Erzeugen eines Laserstrahls, welcher erforderlich ist, um Haut zu durchlöchern;
einen Adapter (5, 6, 14, 15), eingerichtet am vorderen Ende des den Er:YAG-Laserstrahl erzeugenden Abschnitts, derart, dass der Laserstrahl auf einem Hautdurchlöcherungsabschnitt lokal konzentriert werden kann, und
ein Gehäuse (32) zur Aufnahme des Er:YAG-Laserstrahl erzeugenden Abschnitts und des Adapters hierin,
mit einer Kappe (13) zum Anhaften auf der Haut, welche an dem vorderen Ende des Adapters entfernbar befestigt ist, und
mit einem Körper, welcher ein hohles Inneres hat, sodass der Laserstrahl hindurchgehen kann, wobei ein hinterer Endabschnitt des Körpers an dem vorderen Ende des Adapters entfernbar befestigt ist, und
mit einem Film (12), welcher an dem hinteren Ende des Körpers angebracht ist, um die Dispersion des Bluts, welches zum Zeitpunkt des Einstechens auftritt und die Kontamination des Adapters aufgrund des Rauches zu verhindern,
**dadurch gekennzeichnet, dass** der Körper ein vorderes Ende hat, welches hiervon abgewinkelt und so gestaltet ist, dass es in engem Kontakt mit der Haut stehen kann, und
dass der Film (12) ein Inspektionsfilm ist, welcher von dem Laserstrahl im Einsatz zerrissen werden kann, um eine visuelle Anzeige darüber zu bilden, wann die Hautanhaftkappe verwendet worden ist, um das Problem einer Infektion des Patienten aufgrund einer Wiederverwendung der Hautanhaftkappe zu eliminieren.

2. Vorrichtung für einen schmerzlosen Minimaleinstich nach Anspruch 1, bei welcher der vordere Endabschnitt des Körpers der Hautanhaftkappe (13), welche im Einsatz in engem Kontakt mit der Haut steht, um 68° in Bezug auf den Laserstrahl, welcher durch das Innere des Körpers hindurchgeht, geneigt ist.

3. Vorrichtung für einen schmerzlosen Minimaleinstich nach Anspruch 1 oder Anspruch 2, bei der der Er:YAG-L.aserstrahl erzeugende Abschnitt (1-4, 7-11) aufweist:
A) einen Reflektor (2) mit einem Empfangsraum, welcher an der Innenseite gebildet ist;
B) einen Er:YAG-Kristall (9), welcher in dem Empfangsraum des Reflektors angeordnet ist;
C) einen Totalreflexionsschirm (11), welcher an der hinteren Oberfläche des Er-YAG-Kristalls angeordnet ist;
D) einen semipermeablen Spiegel (7), welcher an dem vorderen Ende des Er-YAG-Kristalls angeordnet ist;
E) eine Blitzlampe (3), welche im Inneren des Empfangsraums derart angebracht ist, dass sie in einem bestimmten Intervall von dem Er:YAG-Kristall beabstandet ist;
F) Sicherungsdeckel (1, 4) für die vordere und hintere Oberfläche, welche entsprechend an dem vorderen und an dem hinteren Ende des Reflektors angebracht sind, so dass beide Enden des Er-YAG-Kristalls und die Blitzlampe getragen werden können; und
G) einen Sicherungshalter (8, 10) an der vorderen und hinteren Oberfläche, welcher in die Außenseite beider Seiten des vorderen und des hinteren Endes des Reflektors eingesetzt ist, um einstückig den die vordere und die hintere Oberfläche sichernden Deckel und den Reflektor zu sichern.

4. Vorrichtung für einen schmerzlosen Minimaleinstich nach Anspruch 1 oder Anspruch 2, bei welcher der Adapter (5, 6, 14, 15) folgende Merkmale aufweist:
A) einen Fokussierlinsenhalter (6), welcher mit einem seiner Enden in den Sicherungshalter der vorderen Oberfläche des einen Er:YAG-Laserstrahl generierenden Abschnitts eingesetzt ist;
B) eine Fokussierlinse (5), welche in einen inneren Durchgang des Fokussierlinsenhalters eingesetzt ist, so dass der Laserstrahl auf einem Hautdurchlöcherungsabschnitt konzentriert werden kann;
C) eine Verbindungsvorrichtung (14), welche an dem vorderen Ende des Fokussierlinsenhalters angebracht ist; und
D) eine Pufferfeder (15), welche zwischen der Verbindungseinrichtung und dem Fokussierlinsenhalter gesetzt ist.

5. Blutzuckermessgerät mit folgenden Merkmalen:
eine Vorrichtung für einen schmerzlosen Minimaleinstich nach einem der Ansprüche 1 bis 4;
einem Verbinder (25) zum Einsetzen eines Blutzuckersensorstreifens (24);
eine Blutzuckermessschaltung (23); und
eine Einrichtung (26) zum Anzeigen eines gemessenen Blutzucksnrverts.

6. Blutzuckermessgerät nach Anspruch 5, mit einem Programm zum Aufnehmen/Handhaben von Ergebnissen der Blutzuckermessung, welcher in einer Mikroprozessoreinheit (MPU) (30) installiert ist.

## Revendications

1. Autopiqueur sans douleur minimum utilisant un laser Er:YAG, comportant :
une section de génération de faisceau laser Er:YAG (1-4, 7-11) pour générer un faisceau laser requis pour percer la peau,
un adaptateur (5, 16, 14, 15) installé au niveau de l'extrémité avant de la section de génération de faisceau laser Er:YAG de sorte que le faisceau laser peut être concentré localement sur une partie de perforation de la peau, et
un boîtier (32) pour loger la section de génération de faisceau laser Er:YAG et l'adaptateur à l'intérieur,
comportant en outre un capuchon à adhérence cutanée (13) installé de manière amovible au niveau de l'extrémité avant de l'adaptateur et comportant :
un corps ayant un intérieur creux de sorte que le faisceau laser peut le traverser, une partie d'extrémité arrière du corps étant fixée de manière amovible à l'extrémité avant de l'adaptateur, et
un film (12) installé au niveau de l'extrémité arrière du corps pour empêcher la dispersion du sang qui survient au moment de la piqûre et la contamination de l'adaptateur due à la fumée,
**caractérise en ce que** le corps dispose d'une extrémité avant correspondante qui est inclinée et mise en forme de sorte qu'elle peut être en contact étroit avec la peau, et
le film (12) est un film d'inspection susceptible de se rompre en utilisation par l'intermédiaire du faisceau laser afin de fournir une indication visuelle du moment auquel le capuchon à adhérence cutanée a été utilisé, de manière à éliminer le problème d'infection du patient due à la réutilisation du capuchon à adhérence cutanée.

2. Autopiqueur sans douleur minimum selon la revendication 1, dans lequel la partie d'extrémité avant du corps du capuchon à adhérence cutanée (13), lequel en utilisation est en contact étroit avec la peau, est inclinée de 68° par rapport au faisceau laser traversant l'intérieur du corps.

3. Autopiqueur sans douleur minimum selon la revendication 1 ou la revendication 2, dans lequel la section de génération de faisceau laser Er:YAG (1-4, 7-11) inclut :
A) un réflecteur (2) ayant un espace de réception formée à l'intérieur,
B) un cristal Er:YAG (9) disposé dans l'espace de réception du réflecteur,
C) un miroir à réflexion totale (11) installé au niveau de la surface arrière du cristal Er:YAG,
D) un miroir semi-perméable (7) installé au niveau de l'extrémité avant du cristal Er:YAG,
E) une lampe flash (3) installée à l'intérieur de l'espace de réception de manière à être espacée d'un intervalle particulier par rapport au cristal Er:YAG,
F) des couvercles de fixation de surface avant et arrière (1, 4) respectivement installés au niveau des deux extrémités avant et arrière du déflecteur de sorte que les deux extrémités du cristal Er:YAG et de la lampe flash peuvent être supportées, et
G) un support de fixation de surface avant et arrière (8, 10) ingéré à l'extérieur des deux côtés des extrémités avant et arrière du réflecteur pour fixer intégralement la surface avant et arrière fixant le couvercle et le réflecteur.

4. Autopiqueur sans douleur minimum selon la revendication 1 ou la revendication 2, dans lequel l'adaptateur (5, 6, 14, 15) inclut :
A) un support de lentille de focalisation (6) inséré au niveau d'une extrémité correspondante dans le support de fixation de surface avant de la section de génération de faisceau laser Er:YAG,
B) une lentille de focalisation (5) insérée dans un passage inférieur du support de lentille de focalisation de sorte que le faisceau laser peut être concentré sur une partie de perforation de la peau,
C) un dispositif de connexion (14) installé au niveau de l'extrémité avant du support de lentille de focalisation, et
D) un ressort amortisseur (15) intercalé entre le dispositif de connexion et le support de lentille de focalisation.

5. Lecteur de glycémie comportant :
un autopiqueur sans douleur minimum selon l'une quelconque des revendications 1 à 4,
un connecteur (25) pour insérer une bande de détection de la glycémie (24),
un circuit de mesure de la glycémie (23), et
des moyens (26) pour afficher une valeur de glycémie mesurée.

6. Lecteur de glycémie selon la revendication 5, incluant en outre un programme pour enregistrer/gérer des résultants de la mesure de glycémie installé dans une unité de microprocesseur (MPU) (30).
